# EUROPEAN PATENT APPLICATION

(11) **EP 1 806 224 A1**
(43) Date of publication of application: **11.07.2007**
(21) Application number: 06256222.8
(22) Date of filing: 06.12.2006
(51) Int. Cl.: B32B 27/00

(54) **A hybrid composite for biological tissue interface devices**

(30) Priority: 07.12.2005 US 296605
(71) Applicant: Cordis Corporation, Miami Lakes, FL 33014 (US)
(72) Inventor: Maghribi, Mariam, Fremont, CA 94538 (US)
(74) Representative: Belcher, Simon James

(57) **Abstract**

The present invention provides a hybrid composite for medical devices that interface with biological tissues. The hybrid composite comprises at least one first layer of conformable polymeric material, a second layer of insulating polymeric material, and one or more active components and/or one or more passive components, wherein the one or more active components and/or the one or more passive components are partially or completely embedded in the first layer of conformable polymeric material or the second layer of insulating polymeric material. Preferably, the conformable polymeric material is an elastomer, a hydrogel or a biodissolvable polymer and the insulating polymeric material is parylene or silicon carbide. A method of forming the inventive hybrid composite is also provided.

## Description

The present invention relates to a hybrid composite for medical devices that interface with biological tissues and a method of forming the hybrid composite.

Medical devices, particularly implantable medical devices and external medical devices that need to conform to the anatomy and have electrical components therein (e.g., a patch-like device), have been widely used to replace malfunctioning biological structures or treat diseases associated therewith. Generally, implantable medical devices can be categorized as passive or active devices. Most passive implantable devices are structural devices (e.g., artificial joints and vascular grafts). Active implantable devices are medical implants that depend for their operation on a source of energy other than energy generated by the human body or gravity. A prime example of an active implantable medical device is a cardiac pacemaker. A cardiac pacemaker is typically comprised of stimulation electrodes tethered from a rigid titanium canister that houses the circuitry and power source for protection from biodegradation.

For a cardiac pacemaker, it is still feasible to house the circuitry and power source in a rigid canister separately from the electrode. However, tremendous complexity is added when developing a closed-loop active medical implant, which is a system that enables the device to sense, interpret and treat a medical condition without human intervention. Due to the large quantity of information that must be captured, processed, and transmitted in real-time from the surrounding environment to the closed-loop active implanted device, batteries are no longer a sufficient power supply and must be replaced by a radio frequency (RF) wireless inductive link that transmits both signal and power directly to the implant. Besides the requirement of sophisticated data acquisition and power generating components, the size and shape of medical implants are often dictated by anatomical space constraints.

One major challenge for developing an implantable medical device is the lack of appropriate material. The biostability and biocompatibility of the implant materials are critical for the use of implantable medical devices. Since medical implants are intended for prolonged or permanent use and directly interface with body tissue, body fluids, electrolytes, proteins, enzymes, lipids, and other biological molecules, the materials used for the construction of medical implants must meet stringent biological and physical requirements. Generally, such materials are required to (1) be conformable, i.e., conform to the biological structure without inducing detrimental stress, (2) be robust, i.e., withstand handling during fabrication and implantation, (3) be chemically inert to body tissue and body fluids, and (4) be dielectric, thereby provide electrical insulation to protect tissue from active elements. In addition, it is particularly desirable that materials used for active implantable medical device are capable of interfacing with an integrated circuit (IC) chip and supporting electronics to receive power and data wirelessly to allow for complete system integration.

Materials commonly used for fabricating medical implants include polymeric materials, for example, silicon-based polymers, polyurethanes, polyimides, hydrogels, and biodissolvable polymers. Polydimethylsiloxane (PDMS) is the most widely used silicon-based organic polymer, and is particularly known for its unusual rheological properties. Due to its viscoelastic properties, PDMS coatings are soft and conformable. Since it is important to avoid having sharp edges on implantable devices that might damage surrounding tissues, the conformability of PDMS is highly desirable for interfacing with biological tissues. Further, PDMS is biocompatible and can be processed at low-temperature. Another widely used coating material is hydrogel. Hydrogel hydrates and swells under physiological conditions forming a lubricious and hemocompatible layer. Thus, a hydrogel coating can reduce injury or inflammation of mucous membranes caused by uncoated medical devices during the use or operation thereof. However, PDMS and hydrogel are both permeable to water vapor which can be detrimental to implantable medical devices, particularly active electronic devices. There are also other polymeric materials which have certain desirable properties to be used on medical devices, but are not stable under physiological conditions.

Vapor deposited polymers, such as parylene and silicon carbide, are used to protect a wide variety of mechanical devices. Parylene is an outstanding barrier which has very low permeability to both vapors and liquids. Parylene also provides excellent corrosion resistance and exhibits superior dielectric strength. Parylene has high tensile and yield strength, thereby is mechanically robust. Thus, parylene offers the insulation and robustness needed for a biomedical surface. Further, parylene coatings are completely conformal of uniform thickness and pinhole free, which is achieved by a unique vapor deposition polymerization process. Silicon carbide has high thermal conductivity and high electric field breakdown strength, and is highly inert as well. However, vapor deposited polymers, such as parylene and silicon carbide, are not as viscoelastic as PDMS or other elastic polymers, and thus they do not provide sufficient softness and conformability required for interfacing with biological tissues.

Thus, there remains a need for a material for implantable medical devices that simultaneously provides conformability, insulation, robustness, and completely conformal of uniform thickness. It is also desirable that such a material can interface with an IC chip and support electronics to receive power and data wirelessly so as to allow a complete system integration for active implantable medical devices.

Accordingly, the present invention provides a hybrid composite for medical devices that interfaces with biological tissues. Specifically, the hybrid composite of the present invention comprises at least one first layer of conformable polymeric material, a second layer of insulating polymeric material on the first layer of conformable polymeric material, and one or more active and/or passive components, wherein the one or more active and/or passive components are partially or completely embedded in the first layer of conformable polymeric material or the second layer of insulating polymeric material. Preferably, the conformable polymeric material is an elastomer, a hydrogel or a biodissolvable polymer and the insulating polymeric material is a parylene or silicon carbide.

In another aspect, the present invention is directed to a method of forming a hybrid composite, the method comprising: providing a substrate; applying a layer of conformable polymeric material on at least one surface of the substrate; partially curing the layer of conformable polymeric material; applying an insulating polymeric material on the partially cured layer of conformable polymeric material to form a layer of insulating polymeric material; applying one or more active and/or passive components on the layer of insulating polymeric material; and partially or completely passivating the one or more active and/or passive components with the insulating polymeric material.

Embodiments of the invention will now be described by way of example, with reference to the accompanying drawings, in which:

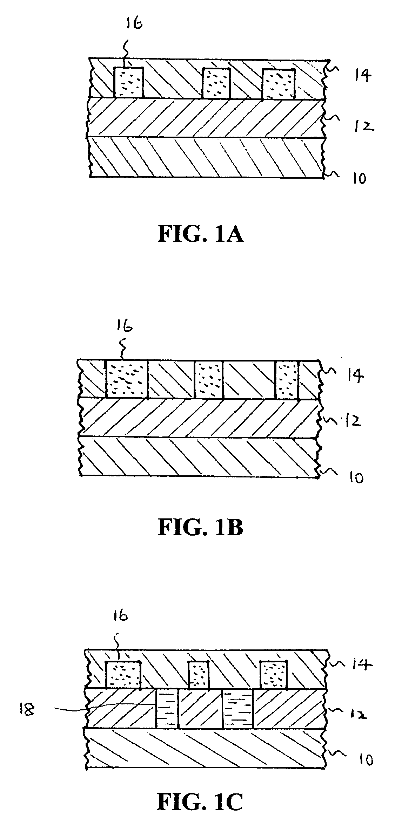

FIG. 1A is a pictorial illustration of one embodiment of a hybrid composite wherein the patterned metallization is completely embedded in the insulating polymeric material.
FIG. 1B is a pictorial illustration of one embodiment of a hybrid composite wherein the patterned metallization is partially embedded in the insulating polymeric material.
FIG. 1C is a pictorial illustration of one embodiment of a hybrid composite wherein the patterned metallization is encapsulated in the insulating polymeric material and the layer of conformable polymeric material has reservoirs incorporated therein.

The present invention provides a hybrid composite material for medical devices. Specifically, the hybrid composite of the present invention comprises at least one first layer of conformable polymeric material, a second layer of insulating polymeric material, and one or more active components and/or one or more passive components, wherein the one or more active components and/or the one or more passive components are partially or completely embedded in the first layer of conformable polymeric material or the second layer of insulating polymeric material. By "active components", it is meant components that require external and/or internal power sources for actuation. By "passive components", it is meant components that do not require any power source for actuation.

In the present invention, the conformable polymeric material is preferably an elastomer, a hydrogel, or a biodissolvable polymer. By "elastomer", it is meant a polymer having the elastic properties of natural rubber. Examples of elastomeric polymers include, but are not limited to: organopolysiloxane, polyutherane, and nitrile rubber. Organopolysiloxane and polyutherane are the preferred elastomers of the present invention, with organopolysiloxane as the more preferred. By "hydrogel", it is meant a polymeric colloidal gel in which water is the dispersion medium. Examples of hydrogels include, but are not limited to: carboxymethyl cellulose. By "biodissolvable polymer", it is meant a polymer that can be degraded or decomposed by a biological process, as by the action of bacterial, plant, or animal. Examples of biodissolvable polymers include, but are not limited to: polyvinyl pyrrolidone, polyethylene glycol, polyethylene oxide, polyvinyl alcohol, polyglycol lactic acid, polylactic acid, polycaprolactone, and polyamino acid. Biodissolvable polymers are also known as bioerodible, bioabsorbable, or biodegradable polymers. In one embodiment of the present invention, the layer of conformable polymeric material is patterned to have vias, channels, reservoirs, connecting ports valves, or grooves incorporated therein. Other conformable polymeric materials suitable for the present invention include, but are not limited to: collagen, chitin, alginate, and other analogous biomaterials.

The term "organopolysiloxane" as used herein denotes a polysiloxane having organic side groups attached to the silicon atom of a silicon-oxygen backbone. Preferably, an organopolysiloxane has the following general formula: 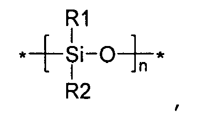 wherein n is an integer; and R1 and R2 are the same or different, and are a hydrogen atom, an alkyl group having 1 to 8 carbon atoms, or an aryl group, provided that R1 and R2 are not both hydrogen. The alkyl group may be a straight, branched, or cyclic alkyl. The term "aryl" is used herein to denote a univalent organic group derived from an aromatic hydrocarbon by the removal of one hydrogen atom. Examples of aryl include, but are not limited to: phenyl, naphthyl, and the like. Preferably, the aryl group is phenyl.

Organopolysiloxanes are also known in the art as silicon-based organic polymers, silicone elastomers, or silicone rubbers. Examples of various organopolysiloxanes that are contemplated by the present invention include, but are not limited to: polydimethysiloxane. In one preferred embodiment of the present invention, the first layer of a biocompatible polymeric material is a layer of polydimethylsiloxane (PDMS). The chemical formula for PDMS is (CH₃)₃SiO[SiO(CH₃)₂]ₙSi(CH₃)₃, where n is an integer.

Organopolysiloxanes have quite flexible polymer backbones (or chains) due to their siloxane linkage. Such flexible chains become loosely entangled when the molecular weight is high, which results in a high level of viscoelasticity. For example, PDMS is particularly known for its unusual rheological properties. By "high molecular weight of organopolysiloxanes", it is meant a molecular weight of about 50K Daltons or above.

Organopolysiloxanes have been widely used as lubricants, adhesives, coatings, and breast implants. The methods of preparing organopolysiloxanes are well known in the art. Organopolysiloxanes can be applied on the surface of a substrate by a conventional coating method, such as spin, cast, mold, spray, or dip.

The thickness of the first layer of conformable polymeric material formed on the article may vary depending on the process used in forming said layer as well as the intended use thereof. Typically, and for a biological tissue interface device, the layer of conformable polymeric material has a thickness from about 1µm to about 10mm with a thickness from about 10µm to about 200µm being more typical.

In the present invention, the insulating polymeric material is preferably a parylene, silicon carbide or other vapor-deposited insulating polymers.

Parylene is not manufactured or sold as a polymer, but rather is produced by vapor-phase deposition and polymerization of para-xylylene or substituted derivatives thereof. Unlike dip or spray coatings, the vacuum-deposited coating is pinhole free and completely conformal of uniform thickness over edges, points, crevices and exposed internal areas. Parylene is also lubricious and resistant to chemicals, gas, and moisture. In addition, parylene has superior dielectric strength and excellent mechanical strength. The parylene coating process is well known to one skilled in the art.

There are four types of parylene variants, namely, parylene N, parylene C, parylene D, and parylene F. Parylene N is poly-para-xylylene, which exhibits a very low dissipation factor, high dielectric strength, and a dielectric constant invariant with frequency. This form has the highest penetrating power of all the parylenes. Parylene C is poly-monochloro-para-xylylene, which has excellent barrier properties. Parylene C offers significantly lower permeability to moisture and gases, while retaining excellent electrical properties. Parylene D is poly-dichloro-para-xylylene, which is similar in properties to parylene C with the added ability to withstand higher use temperatures. When the insulating polymeric material is a parylene, parylene N, parylene C, parylene D, or a mixture thereof may be employed. Parylene F is a fluorinated parylene with properties analogous to Teflon^{®}.

Silicon carbide, also known as moissanite, is a ceramic compound of silicon and carbon. Silicon carbide of the present invention is preferably made by a chemical vapor deposition process. Silicon carbide has high thermal conductivity and high electric field breakdown strength, and is highly inert as well. Silicon carbide is also a material with excellent mechanical durability.

The thickness of the second layer of insulating polymeric material may vary depending on the process used in forming said layer as well as the intended use thereof. Typically, and for biological tissue interface device, the layer of insulating polymeric material has a thickness from about 1nm to about 50µm, with a thickness from about 5nm to about 10µm being more typical.

In the present invention, the one or more active components may be any electrical components. Preferably, the one or more active components of the present invention include, but are not limited to: patterned metallizations, microelectrode arrays, chemical sensors, biological sensors, electrical sensors, integrated circuits, transformers, transistors, and combinations thereof. These elements are typically formed by conventional deposition processes and patterned by lithography and etching. More recently, MEMS technology is used in forming these elements. Metals suitable for the patterned metallization of the present invention include, but are not limited to: Ti, Cr, Au, Ag, Pt, Ir, Ni, Zn, and alloys thereof. Carbon may also be used in the patterned metallization of the present invention. Preferably, the patterned metallization of the present invention comprises a carbon, a metal as described above, or a combination thereof. The one or more passive components of the present invention include, but are not limited to: via, channel, reservoir, connecting port, valve, plug, groove, and a combination thereof. In the present invention, the one or more active components or the one or more passive components are partially or completely embedded in the first layer of conformable polymeric material or the second layer of insulating polymeric material.

In one preferred embodiment of the inventive hybrid composite, the one or more active components are partially or completely embedded in the second layer of insulating polymeric material and are not in direct contact with the first layer of conformable polymeric material. That is, the one or more active components may be encapsulated in the second layer of insulating polymeric material, or the one or more active components may be partially embedded in the second layer of insulating polymeric material exposing only the areas needed for electrical contact. FIGS. 1A-1C show embodiments of the inventive hybrid composite wherein the active component is a patterned metallization not in direct contact with the first layer of conformable polymeric material. FIG. 1A shows one embodiment of the present invention, wherein the patterned metallization is completely embedded, i.e., encapsulated, in the insulating polymeric material. FIG. 1B shows another embodiment of the present invention wherein the patterned metallization is partially embedded in the insulating polymeric material exposing the surface area of the patterned metallization for electrical contact. FIG. 1C shows yet another embodiment of the present invention wherein the patterned metallization is encapsulated in the insulating polymeric material and the layer of conformable polymeric material has reservoirs incorporated therein. In these drawings, reference number 10 denotes a substrate or a portion of a substrate, 12 denotes a layer of conformable polymeric material, 14 denotes a layer of insulating polymeric material, 16 denotes a patterned metallization, and 18 denotes reservoirs.

In another preferred embodiment of the present invention, the hybrid composite comprises at least one first layer of polydimethylsiloxane, a second layer of parylene, and a microelectrode array; wherein the microelectrode array is encapsulated in the second layer of parylene and is not in direct contact with the first layer of polydimethylsiloxane.

The hybrid composite of the present invention synergistically combines the desirable characteristics of conformable polymeric material and insulating polymeric material, and thereby is ideal for implantable medical devices, which directly interface with intricate biological systems. Particularly, the numerous bonding capabilities of the inventive hybrid composite enable the integration of IC and power, which is required in the manufacture of close-loop active implantable devices. When the conformable polymeric material of the first layer of the inventive hybrid composite is an organopolysiloxane and the insulating polymeric material of the second layer of the inventive hybrid composite is parylene, the layer of organopolysiloxane provides the softness and conformability needed to interface with the surrounding tissues, while the layer of parylene provides the insulation and robustness needed to protect the active medical implants. Both of organopolysiloxane and parylene are biocompatible materials that have been used in many medical devices, such as contact lens, breast implants, and catherter tubing. Since organopolysiloxane and parylene have been used successfully in surface micromachining application in which successive layers are deposited and patterned to create micromechanical structures, the inventive hybrid composite accommodates the integration of microfabrication techniques and the manufacture of medical devices, and thereby provides the capacity of batch fabrication processing which leads to lower cost and high yield. In fact, the material cost per weight of the inventive hybrid composite is less than that of silicon or glass.

The inventive hybrid composite has both the compliance to match the mechanical properties of surrounding tissues and sufficient strength to support mechanical loads. That is, the inventive hybrid composite has not only the flexibility and stretchability to move with the body, but also the strength to serve as a substrate for supporting other implanted components. Young's modulus, also known as the modulus of elasticity, is a measure of the stiffness of a given material. The SI unit of modulus of elasticity is the pascal (Pa). Table 1 provides a comparison of Young's modulus of various materials.

**Table 1. Comparison of Young's Modulus of Various Materials (Dissertation of Mariam Maghribi, 2003, University of California - Davis)**

| Material | Young's Modulus |
|---|---|
| Silicon | 165 GPa |
| Polyimide | 1.3 - 4 GPa |
| Parylene | 3.2 GPa |
| PDMS | 750 kPa - 3 Mpa |
| Hydrogel | 160 - 290 kPa |
| Brain Tissue | 66 - 250 kPa |

The mechanical properties of the inventive hybrid composite can be modified via adjusting the ratio of the first layer conformable polymeric material (e.g., organopolysiloxane) to the second layer of insulating polymeric material (e.g., parylene). For example, the mechanical properties of the inventive hybrid composite can be modified via adjusting the thickness of the first layer conformable polymeric material and the second layer of insulating polymeric material. Typically, the stiffness of the inventive hybrid composite increases when thickness of the second layer of insulating polymeric material increases. Thus, the first layer conformable polymeric material to the second layer of insulating polymeric material ratio can be tailored according to the intended use and the application need. Specifically, the thickness of the first layer of conformable polymeric material and the second layer of insulting polymeric material can be independently adjusted. The thickness ranges provided above can be used for this adjustment. Further, the stiffness of the first layer of conformable polymeric material can be tuned through cure temperature and cure time.

The inventive hybrid composite can be in various shapes. The shape of the inventive hybrid composite can be adjusted according to the contour of the substrate on which it is applied.

The inventive hybrid composite is suitable to be applied on or integrated into medical implants or a component thereof, particularly an active implantable medical device or a component thereof, because the inventive hybrid composite is capable of interfacing to an integrated circuit chip and supporting electronics to receive power and data wirelessly. Examples of implantable medical devices suitable for the present invention include, but are not limited to: cochlear implants, retinal implants, gastric bands, neurostimulation devices, muscular stimulation devices, implantable drug delivery devices, intraocular devices, and various active implantable medical devices. When integrated into an active implantable medical device, the inventive hybrid composite may receive electrical stimulation from devices that are electrically connected to or isolated from the tissues in the forms that include, but are not limited to: currents or voltages applied directly to the tissue; static electric fields, static magnetic fields, or a combination thereof; time-varying electric or magnetic fields, or a combination thereof; and electromagnetic fields such as radio waves, microwaves, infrared, visible and ultraviolet light. Various devices that may be used to deliver the electrical energy to the inventive hybrid composite include, but are not limited to: antennas such as coils, inductors, dipole, log-periodic; field-emission devices; electrodes and electrode arrays. Any of these electrical energy delivering devices may be deployed in 3D configurations and matrices that allow electrical fields, voltages and currents through and across tissues to be changed and optimized. Electrical energy delivery may be time varying with a profile that includes, but is not limited to: sinusoidal, square-wave, on/off/positive/negative pulsing, and any combination thereof. Ultrasonic energy may be applied using sources that include, but are limited to: piezolelectric sources, electrostrictive sources, electromagnetic sources, ultraviolet light, infrared light, and microwaves.

In another aspect of the present invention, the method of forming a hybrid composite comprises the steps of: providing a substrate; applying a layer of conformable polymeric material on at least one surface of the substrate; partially curing the layer of conformable polymeric material; applying insulating polymeric material on the partially cured layer of conformable polymeric material to form a layer of insulating polymeric material; applying one or more active and/or passive components on the layer of insulating polymeric material; and partially or completely passivating the one or more active and/or passive components with the insulating material.

In the method described above, conformable polymeric material is first prepared from a known precursor of the polymeric material via methods known to those skilled in the art. Preferably, the conformable polymeric material is an organopolysiloxane. Then, the conformable polymeric material is deposited on at least one surface of an article by known means, to form a layer of the polymeric material. The conformable polymeric material can be spun, cast, molded, sprayed, or dipped on the surface of the article at desired thickness and shape. The thickness of polymeric material can be tailored by varying the ratio of the curing agent to the resin, or by adding an organic solvent. In one embodiment of the present invention, the organic solvent is toluene. In a more preferred embodiment of the present invention, the conformable polymeric material is PDMS.

In one embodiment of the present invention, the layer of conformable polymeric material is further patterned to incorporate one or more passive components via lithographic techniques known to one skilled the art. The one or more passive components suitable for the present invention include, but are not limited to: via, channel, reservoir, connecting port, valve, plug, groove, and a combination thereof.

Next, the resulting layer of the conformable polymeric material is partially cured, i.e., incompletely cured. The partial or incomplete cure of the conformable polymeric material is an essential step of the inventive method because partial or incomplete cure retains the freshness of the conformable polymeric material, and thus improves the adhesion of the insulating polymeric material deposited thereon. By "partial cure" or "incomplete cure", it is meant a curing process wherein the cure time is substantially less than the normal or standard cure time. Preferably, the cure time for such a partial or incomplete cure is equal to or less than about 50% of the normal or standard cure time. The cure temperature for such a partial or incomplete cure is equal to or lower than the normal or standard cure temperature. In one preferred embodiment of the present invention, the polymeric material is PDMS. In the step of partial cure, PDMS is partially cured for about 20 to 60 minutes at about 60° to 70°C, while the standard curing time for PDMS is about 4 hours at about 60° to 70°C.

After the partial cure step, the insulating polymeric material is deposited on the layer of the conformable polymeric material forming a layer of insulating polymeric material. Preferably, the insulating polymeric material is parylene, silicon carbide or other vapor deposited insulating polymers. Preferably, the insulating polymeric material is deposited via a vapor deposited method. For example, parylene deposition typically takes place in vacuum using a gas phase process that converts a solid crystalline dimer to a gaseous form, then to a stable monomeric gas, and finally to a polymer.

In one embodiment of the present invention, the surface of the layer of insulating polymeric material is rinsed with organic solvents following the deposition of the insulating polymeric material. Suitable organic solvents for rinsing the surface of the layer of insulating polymeric material include, but are not limited to: ethanol and isopropynol. The rinsed surface of the layer of insulating polymeric material is then dried with clean air, nitrogen or inert gases. Next the surface of the layer of insulating polymeric material is treated with oxygen plasma. Typically, the treatment of oxygen plasma is at about 100 to 150 watts for about 1 to 10 minutes depending on the plasma system.

Then, one or more active and/or passive components are applied onto the surface of the layer of insulating polymeric material via the methods known to one skilled the art. The one or more passive components of the present invention include, but are not limited to: via, channel, reservoir, connecting port, valve, plug, groove, and a combination thereof. The one or more active components of the present invention may be any electrical components. Examples of the one or more active components include, but are not limited to: patterned metallization, microelectrode array, chemical sensor, biological sensor, circuit, and a combination thereof. In a preferred embodiment of the present invention, the one or more active components are a patterned metallization. The patterned metallization comprises metals that include, but are not limited to: Ti, Cr, Au, Ag, Pt, Ir, Ni, Zn, and a combination thereof. Carbon may also be used in the patterned metallization of the present invention. Preferably, the patterned metallization of the present invention comprises a carbon, a metal as described above, or a combination thereof. Suitable methods for applying the patterned metallization include, but are not limited to: vapor deposition of metals, screen printing conductive inks, integration of chemically etched metals, chemically etching metals onto the insulting polymeric material, integration of laser etched metals, and laser etching metals directly onto the insulting polymeric material. Specifically, the method of vapor deposition of metals includes, but is not limited to: sputter deposition, electron-beam deposition, and thermal evaporation.

The one or more active and/or passive components are then passivated partially or completely for protection. That is, the insulating polymeric material deposited to form the layer of insulating polymeric material is applied onto the one or more active and/or passive components and portions of the layer of insulating polymeric material not covered by the one or more active and/or passive components. In one embodiment of the present invention, the one or more active and/or passive components are encapsulated into the insulating polymeric material. In another embodiment of the present invention, the one or more active and/or passive components are partially covered by the insulating polymeric material exposing only the areas needed for electrical contact. In other words, the one or more active and/or passive components may be embedded partially or completely in the insulating polymeric material.

The inventive hybrid composite can further be patterned using lithographic techniques known to one skilled the art.

## Claims

1. A hybrid composite comprising at least one first layer of conformable polymeric material, a second layer of insulating polymeric material on the first layer of conformable polymeric material, and one or more active and/or passive components, wherein the one or more active and/or passive components are partially or completely embedded in the first layer of conformable polymeric material or the second layer of insulating polymeric material.

2. The hybrid composite of claim 1, wherein the one or more active components are partially or completely embedded in the second layer of insulating polymeric material and are not in direct contact with the first layer of conformable polymeric material.

3. The hybrid composite of claim 1, wherein the conformable polymeric material is an elastomer, a hydrogel, or a biodissolvable polymer.

4. The hybrid composite of claim 3, wherein the elastomer is an organopolysiloxane or polyurethane.

5. The hybrid composite of claim 4, wherein the organopolysiloxane is polydimethylsiloxane.

6. The hybrid composite of claim 1, wherein the insulating polymeric material is a parylene or silicon carbide.

7. The hybrid composite of claim 6, wherein the parylene is parylene C, parylene N, parylene D, parylene F, or a mixture thereof.

8. The hybrid composite of claim 1, wherein the first layer of conformable polymeric material has a thickness of about 1 µm to about 10 mm.

9. The hybrid composite of claim 1, wherein the second layer of insulating polymeric material has a thickness of about 1 nm to about 50 µm.

10. The hybrid composite of claim 1 or 2, wherein the one or more active components are selected from the group consisting of patterned metallization, microelectrode array, chemical sensor, biological sensor, electrical sensor, integrated circuit, transformer, transistor, and a combination thereof.

11. The hybrid composite of claim 10, wherein the patterned metallization comprises carbon, a metal selected from the group consisting of Ti, Cr, Au, Ag, Pt, Ir, Ni, Zn, and alloys thereof, or a combination thereof.

12. The hybrid composite of claim 1, wherein the one or more passive components are selected from a group consisting of via, channel, reservoir, connecting port, valve, plug, groove, and a combination thereof.

13. A method of forming a hybrid composite, the method comprising:
providing a substrate;
applying a layer of conformable polymeric material on at least one surface of the substrate;
partially curing the layer of conformable polymeric material;
applying an insulating polymeric material on the partially cured layer of conformable polymeric material to form a layer of insulating polymeric material;
applying one or more active and/or passive components on the layer of insulating polymeric material; and
partially or completely passivating the one or more active and/or passive components with the insulating polymeric material.

14. The method of claim 13, wherein the conformable polymeric material is an elastomer, a hydrogel, or a biodissolvable polymer.

15. The method of claim 14, wherein the elastomer is an organopolysiloxane or polyurethane.

16. The method of claim 15, wherein the organopolysiloxane is polydimethylsiloxane.

17. The method of claim 13, wherein the insulating polymeric material is a parylene or silicon carbide.

18. The method of claim 17, wherein the parylene is parylene C, parylene N, parylene D, parylene F, or a mixture thereof.

19. The method of claim 13, wherein the one or more active components are selected from the group consisting of patterned metallization, microelectrode array, chemical sensor, biological sensor, electrical sensor, integrated circuit, transformer, transistor, and a combination thereof.

20. The method of claim 19, wherein the patterned metallization comprises carbon, a metal selected from the group consisting of Ti, Cr, Au, Ag, Pt, Ir, Ni, Zn, and alloys thereof, or a combination thereof.

21. The method of claim 15, wherein the step of partially curing is a process of curing the layer of organopolysiloxane for an amount of time which is substantially less than the normal or standard time for curing organopolysiloxane.

22. The method of claim 15, wherein the step of partially curing is a process of curing the layer of organopolysiloxane for an amount of time which is equal to or less than 50% of the normal or standard time for curing organopolysiloxane.

23. The method of claim 22, wherein the partial cure is conducted at a temperature of about 60°C to about 70°C for about 20 to about 60 minutes.

24. The method of claim 13, wherein the insulating polymeric material is applied by using a vapor deposition polymerization process.

25. The method of claim 13, wherein the partial or complete passivation of the one or more active components comprises applying the insulating polymeric material on the one or more active components and portions of the layer of insulating polymeric material not covered by the one or more active components to partially or completely embed the one or more active components in the insulating material.

26. The method of claim 13, wherein the layer of insulating polymeric material is treated with oxygen plasma prior to applying one or more active components thereon.

27. The method of claim 13, wherein the layer of conformable polymeric material is patterned to incorporate one or more passive components prior to partial cure of the layer of conformable polymeric material.

28. The method of claim 27, wherein the one or more passive components are selected from the group consisting of via, channel, reservoir, connecting port, valve, plug, groove, and a combination thereof.
